# EUROPEAN PATENT APPLICATION

(11) **EP 2 476 368 A1**
(43) Date of publication of application: **18.07.2012**
(21) Application number: 11000331.6
(22) Date of filing: 17.01.2011
(51) Int. Cl.: A61B 5/00, A61B 1/24

(54) **Intra-oral camera with polarized and unpolarized light**

(71) Applicant: Carestream Health, Inc., Rochester, NY 14608-1733 (US)
(72) Inventor: Rongguang, Liang, Penfield NY 14526 (US)
(74) Representative: Carstens, Dirk Wilhelm

(57) **Abstract**

An intra-oral imaging apparatus having an illumination system directing light symmetrically about an optical axis. A first array includes polychromatic light sources directing light over a first range of angles. A second array includes polychromatic light sources, each light source of the second array paired with a polarizer directing light over a second range of angles that is narrower than the first range of angles. A third array includes UV light sources directing the light over the second range of angles. Collection optics with an imaging lens system is disposed along an optical path direct reflected light to a detector that is disposed to form image data from the reflected light. An analyzer has its transmission axis oriented orthogonally to the polarizer to reduce specular reflection. A long pass filter blocks reflected UV light from the third array light sources.

## Description

### FIELD OF THE INVENTION

The invention relates generally to the field of diagnostic imaging and more particularly relates to an intra-oral camera that provides light sources of multiple types suitable for imaging.

### BACKGROUND OF THE INVENTION

While there have been improvements in detection, treatment, and prevention techniques, dental caries remains a prevalent condition affecting people of all age groups. If not properly and promptly treated, caries can lead to tooth damage and possibly loss of teeth.

Traditional methods for caries detection include visual examination and tactile probing with a sharp dental explorer device, often assisted by radiographic (x-ray) imaging. Detection using these methods can be somewhat subjective, varying in accuracy due to many factors, including practitioner expertise, location of the infected site, extent of infection, viewing conditions, accuracy of x-ray equipment and processing, and other factors. There are also hazards associated with conventional detection techniques, including the risk of damaging weakened teeth and spreading infection with tactile methods as well as exposure to x-ray radiation. By the time caries is evident under visual and tactile examination, the disease can be in an advanced stage, requiring a filling and, if not timely treated, possibly leading to tooth loss. Similarly, oral cancer detection at early stages is a desirable goal.

In response to the need for early detection of these conditions, there has been considerable interest in improved imaging techniques that do not employ ionizing radiation, such as x-rays. Earlier imaging systems employed reflectance of white light for detecting caries and oral cancer conditions. However, these systems met with little success or acceptance among dental professionals, due to relatively poor performance.

Recognizing the difficulties with using white light reflectance, researchers turned to methods that employ fluorescence, caused when teeth are illuminated with high intensity ultraviolet (UV) light. One such technique, termed Quantitative Light-induced Fluorescence (QLF), operates on the principle that sound, healthy tooth enamel yields a higher intensity of fluorescence under excitation from some wavelengths than does de-mineralized enamel that has been damaged by caries infection. The strong correlation between mineral loss and loss of fluorescence for UV light excitation is then used to identify and assess carious areas of the tooth. A different relationship has been found for red light excitation, a region of the spectrum for which bacteria and bacterial by-products in carious regions absorb and fluoresce more pronouncedly than do healthy areas. Similarly, oral cancer lesions respond differently with regard to excitation light than does normal tissue. Quantification of caries based on a digital image of a tooth such as a fluorescence image provides numerical information on the severity of lesion regions and can help dentists make and carry out treatment plans. It can be a useful tool in the longitudinal monitoring of caries for dentists to observe the evolution of each lesion area over time.

Although fluorescence imaging showed some improved accuracy over imaging methods using white light reflectance, however, results have not shown sufficient improvement for wide acceptance.

An improvement over methods using reflectance or fluorescence, described in commonly assigned U.S. Patent Application Publication No. 2008/0056551, combines both the reflectance and fluorescence images of the tooth to detect caries. This method takes advantage of the observed back-scattering, or reflectance, for incipient caries and in combination with fluorescence effects, to provide an improved dental imaging technique to detect caries. The technique, referred to as Fluorescence Imaging with Reflectance Enhancement (FIRE), helps to increase the contrast of images over that of earlier approaches, and also makes it possible to detect incipient caries at stages when preventive measures are likely to take effect. Advantageously, FIRE detection can be accurate at an earlier stage of caries infection and oral cancer than has been exhibited using existing fluorescence approaches that measure fluorescence alone. The application describes a downshifting method to generate the FIRE image from reflectance and fluorescence data.

In addition to image capture directly for diagnostic imaging, various types of intra-oral cameras have also been developed for obtaining images of tooth and soft tissue surfaces within the mouth as a part of more general intra-oral observation. This capability is used, for example, to support periodontics, orthodontics, and cosmetic dentistry.

The illumination requirements for diagnostic imaging conflict with those for intra-oral observation in a number of ways, as demonstrated in the listing of Table 1. While both observation and diagnostic functions use a broadband white light source, for example, conditioning of the light with regard to angle, distance, and uniformity are different. Figure 1A shows illumination distribution at an angle α1 and variable distance D1 for conventional intra-oral imaging. By comparison, Figure 1B shows the narrower illumination distribution angle α2 and fixed distance D2 that is used for diagnostic FIRE imaging.

Conventional imaging is polarization-insensitive, while diagnostic imaging uses polarized light in order to reduce specular reflection. Conventional imaging uses white light only, while diagnostic FIRE imaging uses combined white light and fluorescent images taken in rapid succession. Specular reflection is acceptable for conventional intra-oral imaging, but is undesirable for diagnostic imaging.

Added to the complexity of providing different illumination sources and treatment for observation and diagnostic imaging is the inherent problem of providing an imaging device that is compact enough for maneuvering and accessing each tooth and other areas of the mouth.

In the face of these conflicting illumination requirements and due to the highly compact packaging that is needed, separate imaging devices have been developed for observation and diagnostic imaging functions. This, in turn, sets a high imaging cost to the dental practitioner. Thus, it can be seen that there would be advantages to a single imaging apparatus that supported both conventional intra-oral observation and diagnostic FIRE imaging

**Table 1**

| **Summary of Conflicting Illumination Requirements** | | |
|---|---|---|
| **Feature** | **For conventional intra-oral observation** | **For caries/oral cancer diagnostics** |
| **Light source** | Broadband white light | White light and narrow-band UV light |
| **Specular reflectance** | Not desirable, but acceptable. | Not desirable. |
| **Polarizer** | Not needed. | Required. |
| **Depth of field** | Variable. 10 to 300 mm | Fixed 5-20 mm, nominal |
| **Field of view** | Large, variable. Sufficient for obtaining largest possible image. | Small, predefined (example: 20x15 mm) |
| **Uniformity** | Moderate levels. | Higher level needed for both white and UV light. |
| **Efficiency** | Moderate efficiency acceptable. | High efficiency required. |

### SUMMARY OF THE INVENTION

It is an object of the present invention to advance the art of dental imaging by providing a single imaging apparatus that is capable of providing both conventional intra-oral images and diagnostic images, such as FIRE images.

Another object of the present invention is to provide a compact illumination subsystem that generates light of multiple types and at different angles, suitable for the particular requirements of observation and diagnostic images.

An advantage of the present invention is an illumination arrangement that allows compact packaging for an intra-oral imaging apparatus.

These objects are given only by way of illustrative example, and such objects may be exemplary of one or more embodiments of the invention. Other desirable objectives and advantages inherently achieved by the disclosed invention may occur or become apparent to those skilled in the art. The invention is defined by the appended claims.

According to one aspect of the invention, there is provided an intra-oral imaging apparatus comprising: an illumination system disposed to direct light symmetrically about an optical axis and comprising: a first array comprising a plurality of polychromatic light sources wherein the first array of polychromatic light sources are disposed about the optical axis to direct light over a first range of angles; a second array comprising a plurality of polychromatic light sources wherein the second array of polychromatic light sources are disposed about the optical axis and wherein each light source of the second array is paired with a polarizer to direct light over a second range of angles that is narrower than the first range of angles; a third array comprising a plurality of ultraviolet light sources disposed about the optical axis to direct the light over the second range of angles; and collection optics disposed along an optical path to direct reflected light to a detector that is disposed to form image data from the reflected light, the collection optics comprising: an analyzer having its transmission axis oriented orthogonally to the polarizer to reduce specular reflection; a long pass filter disposed to block reflected ultraviolet light from the third array light sources; an imaging lens system.

Advantageously, the apparatus of the present invention can be implemented in a compact package that can be used for providing different types of images for a patient. The optical system does not require operator reconfiguration when changing from one type of imaging to another.

### BRIEF DESCRIPTION OF THE DRAWINGS

The foregoing and other objects, features, and advantages of the invention will be apparent from the following more particular description of the embodiments of the invention, as illustrated in the accompanying drawings. The elements of the drawings are not necessarily to scale relative to each other.

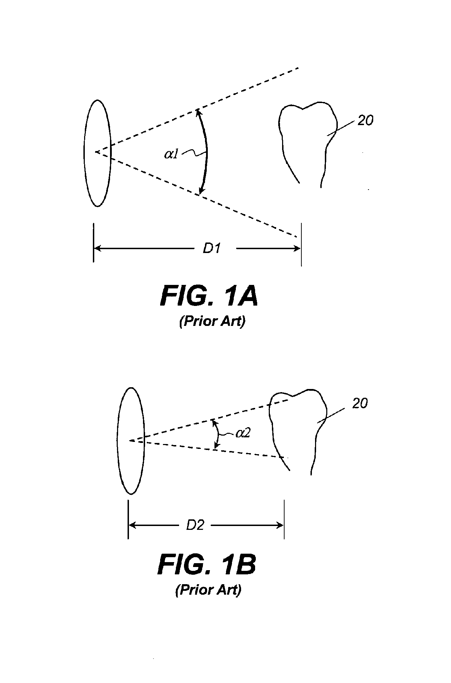

FIG. 1A shows illumination distribution at an angle and variable distance for conventional intra-oral imaging.
FIG. 1B shows the narrower illumination distribution angle and fixed distance that is used for diagnostic imaging.
FIG. 2 shows components of an intra-oral imaging apparatus in one embodiment of the present invention.
FIG. 3 shows a plan view of an illumination system for the intra-oral imaging apparatus.
FIG. 4 is a schematic diagram showing the intra-oral imaging apparatus configured for intra-oral observation.
FIG. 5 is a schematic diagram showing the intra-oral imaging apparatus configured for obtaining fluorescence image data.
FIG. 6 is a schematic diagram showing the intra-oral imaging apparatus configured for obtaining reflectance image data.
FIG. 7 is a side view showing a reflector in one embodiment.
FIG. 8 is a perspective view showing the reflector of FIG. 7 with illumination system components.
FIG. 9 is a schematic diagram showing an alternate embodiment that adds illumination along the center of the field.

### DETAILED DESCRIPTION OF THE INVENTION

The following is a detailed description of the preferred embodiments of the invention, reference being made to the drawings in which the same reference numerals identify the same elements of structure in each of the several figures. Where they are used, the terms "first", "second", and so on, do not necessarily denote any ordinal or priority relation, but may be simply used to more clearly distinguish one element from another.

In the context of the present disclosure, the terms "white light" and "polychromatic light" are considered to be synonymous. A white light source can be a single LED or other source or can be an arrangement with a combination of multiple LEDs or other sources of different colors. Ultraviolet (UV) light, used in FIRE imaging to excite fluorescent response, is generally at wavelengths below about 400 nm.

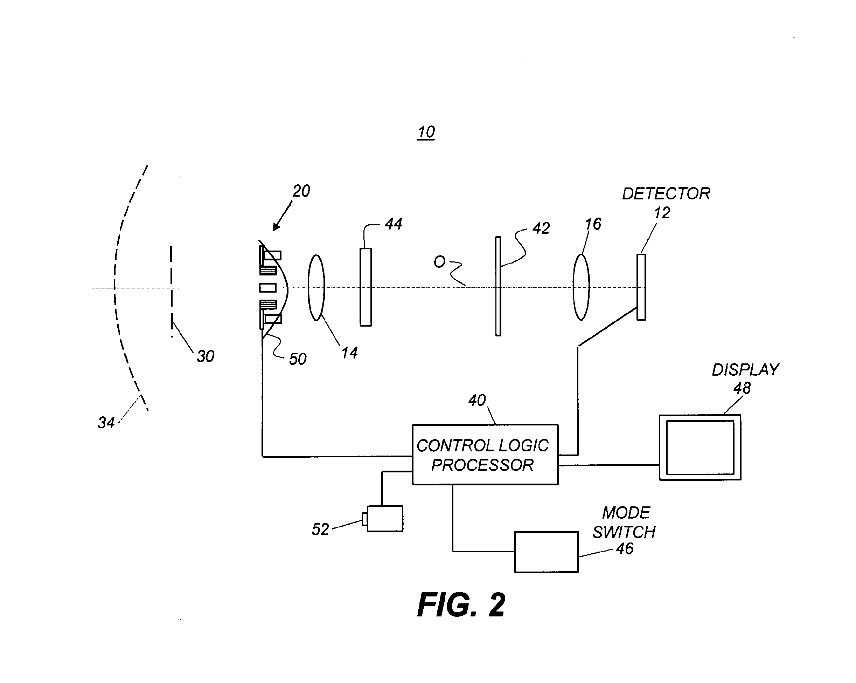

The schematic block diagram of Figure 2 shows components of an intra-oral imaging apparatus 10 that obtains both diagnostic FIRE images, with combined fluorescence and reflectance data, and conventional observation images. An illumination system 20 contains the light sources that are used for each imaging function. For observation imaging, illumination system 20 directs white light at a relatively large angle to an illumination field 34. For caries and oral cancer detection, illumination system 20 directs polarized polychromatic or white light and short-wavelength UV light to a smaller illumination field 30. The short wavelength light is in the UV or near-UV, such as 400 nm wavelength in one embodiment. A reflector 50, described in more detail subsequently, helps to provide the needed light distribution for each of the different illumination sources. Imaging collection optics, shown in Figure 2 as imaging lenses 14 and 16, then direct the resulting image content from the tooth or other structure along an optical axis O to a detector 12, such as a charge-coupled device (CCD) detector, complementary metal-oxide semiconductor (CMOS) detector, or other image sensor array that is energizable for forming an image. A control logic processor 40, which may be packaged together with optics, illumination, and sensing components within intra-oral imaging apparatus 10 or may be external to this device and connected in some manner, controls actuation of light sources in illumination system 20 and acquisition of image data from detector 12, under control of a mode switch 46 or other signal that selects either observational or diagnostic imaging. An optional switch 52 or suitable signal provides the instruction for image capture. Switch 52 is mounted on the body of intra-oral imaging apparatus 10 in one embodiment. The diagnostic image that is formed from the combined polarized white light and UV image data can be stored in a computer-readable storage medium for subsequent display and processing. A display 48 shows the observation or diagnostic image that is obtained.

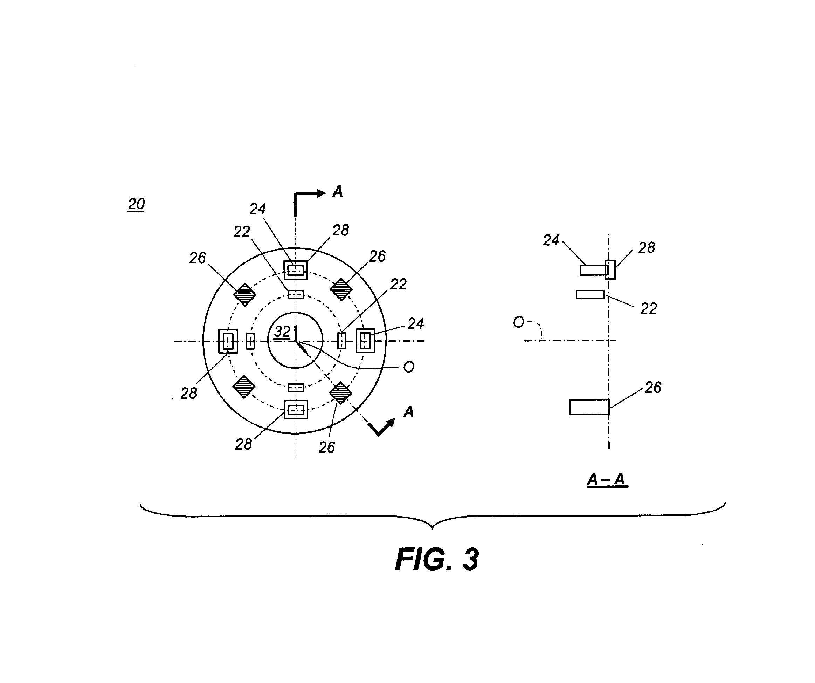

Figure 3 shows a front plan view of illumination system 20 for providing three different types of light, at three different times, and at either of two angular ranges for diagnostic and imaging purposes. View A-A to the right shows the three types of light sources, each type arranged radially as a bank or array of light sources that can be energized as a unit. A first array of polychromatic or white LEDs 22 or other white light sources is provided to support intra-oral observation imaging. The array of LEDs 22 is disposed nearest the optical axis O. A second array of polychromatic or white LEDs 24 provides light for diagnostic FIRE imaging; each white LED 24 is provided with a polarizer 28. A third array of UV LEDs 26 provides excitation light for obtaining the fluorescent component of the FIRE image. An aperture 32 allows reflected and fluorescent light from the tooth or other surface to pass through to collection optics.

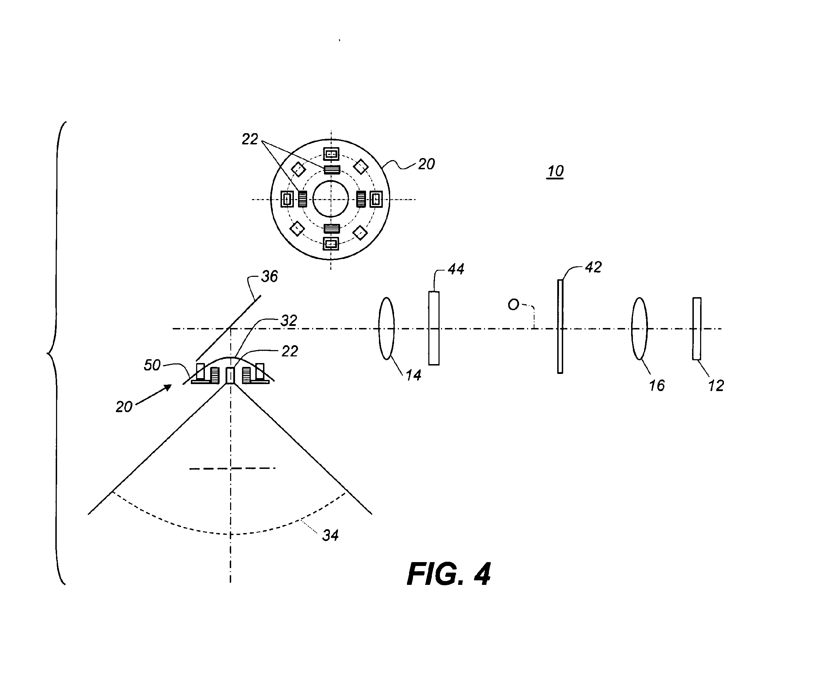

The schematic diagrams of Figures 4, 5, and 6 show the operation of intra-oral imaging apparatus 10 in each imaging mode. For the embodiment in each of these figures, a fold in the optical path is provided using a reflective surface, shown as a fold mirror 36, along optical axis O. This optional fold of the optical axis helps to make intra-oral imaging apparatus 10 easier to use in the patient's mouth. Control logic processor 40, shown previously in Figure 2, is not shown in Figures 4 - 6 for clarity, but would be provided by some type of integrated or external logic processing device, such as a microprocessor or computer, for example. Alternately, control logic processor 40 functions can be distributed among multiple processors, including networked processors, for example.

Figure 4 shows the operation of intra-oral imaging apparatus 10 for conventional intra-oral imaging functions. For this purpose, control logic actuates the array of white LEDs 22 in order to provide illumination along the optical axis O to illumination field 34 over a relatively large range of angles, such as 80 degrees, for example. This arrangement works best for conventional intra-oral viewing, without generating diagnostic data. The white LED 22 light sources are not polarized. Unpolarized light provides the most natural illumination for tooth and tissue surfaces as well as for portions of tooth and tissue just beneath the surface. An analyzer 44 is also positioned in the imaging path, but is not needed for conventional intra-oral examination. Analyzer 44 and a long-pass filter 42 are not needed for conventional viewing and can alternately be switched in or out of the optical path as needed, depending on the imaging function needed at any time.

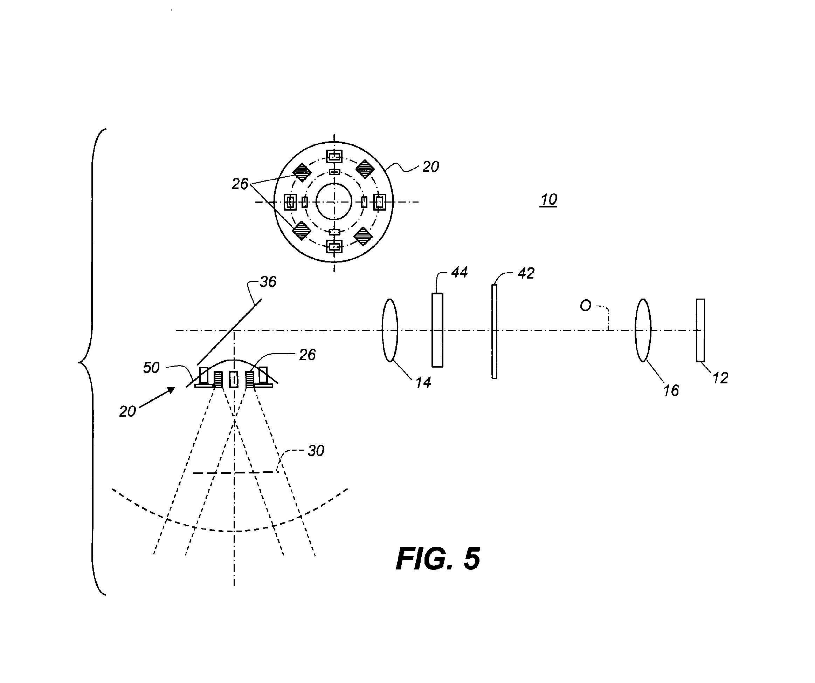

Figure 5 shows the operation of intra-oral imaging apparatus 10 for obtaining a fluorescent image, such as the fluorescent image component of the FIRE image. For this purpose, control logic actuates the array of UV LEDs 26 in order to provide illumination to illumination field 30 over a reduced range of angles, narrower when compared to the range of illumination angles for intra-oral imaging in Figure 4. A long-pass filter 42 is interposed along the optical path for attenuating reflected UV excitation light and passing the emitted fluorescent light, normally in the green wavelengths.

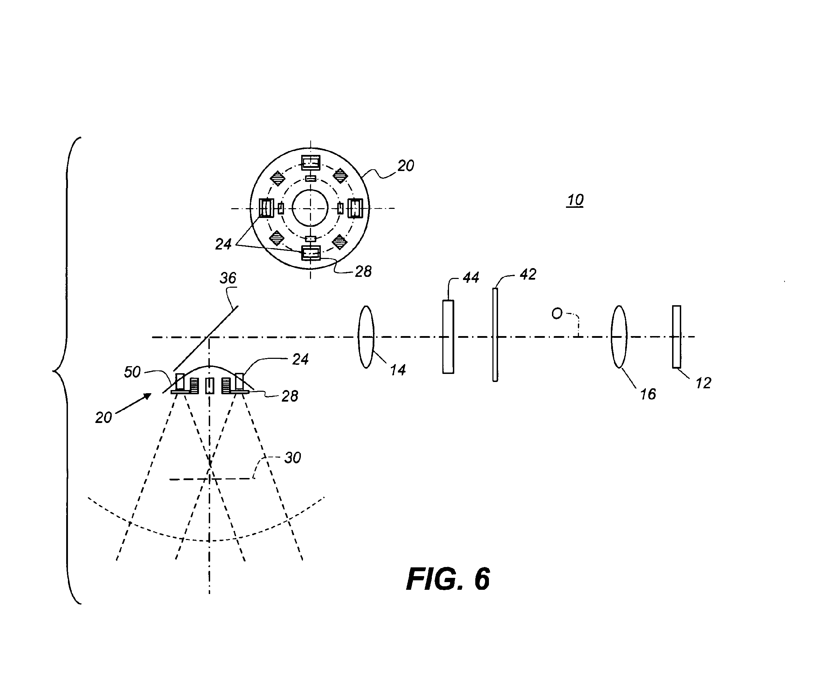

Figure 6 shows the operation of intra-oral imaging apparatus 10 for obtaining a polarized white light image, such as the white light image component of the FIRE image. For this purpose, control logic actuates the array of white LEDs 24 in order to provide polarized illumination to illumination field 30 over a reduced angle, substantially the same angular range used for obtaining the fluorescent image in Figure 5. Each LED 24 is paired with a polarizer 28. An analyzer 44 is interposed along the optical path for obtaining the polarized white light image, helping to eliminate specular reflection.

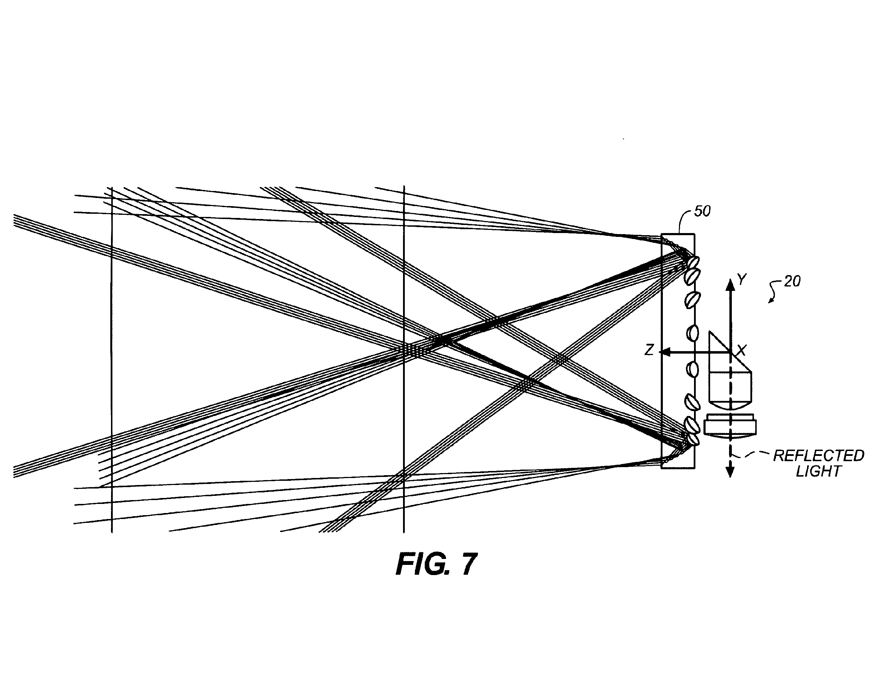

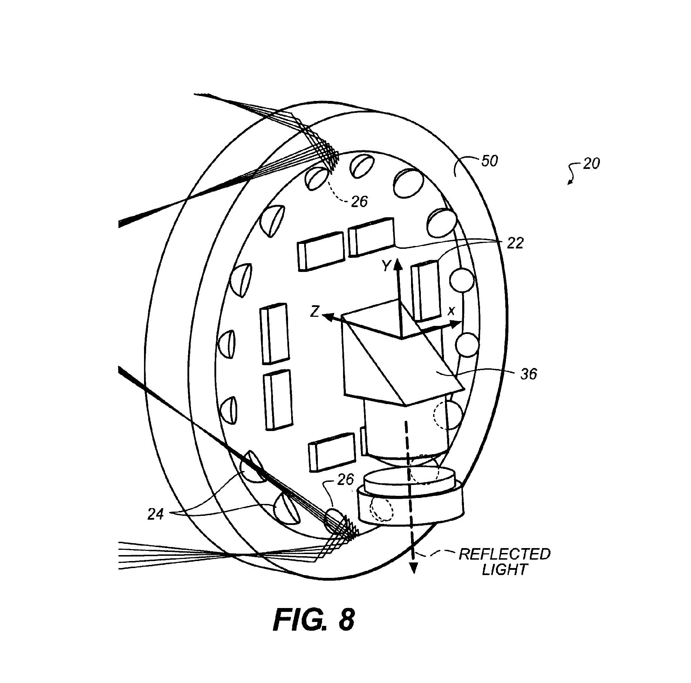

One challenge for design of intra-oral imaging apparatus 10 relates to obtaining the needed illumination distribution and light efficiency from the different arrays of LED sources. The embodiment in Figure 4 may not provide sufficient illumination uniformity and its efficiency can be low. This is because a large portion of light from the LEDs can be directed outside the imaging region and, therefore, lost. Figure 7 shows a side view of reflector 50 that is configured to direct light outward from illumination system 20 at suitable angles. In Figure 7 and the perspective view of Figure 8, the UV light path is represented. It can be seen that reflector 50 is disposed to redirect the UV light outward at narrower angles than the light direction provided from white LEDs 22 that are disposed closer to the center of illumination system 20. In one embodiment, UV LEDs 26 and white LEDs 24 are tilted toward reflector 50 in order to improve the light-directing properties of reflector 50. Reflector 50 can be elliptical, parabolic, or some other suitable shape. Reflector 50 not only improves the illumination uniformity, but also provides a significant increase in light efficiency.

The light sources used in the described embodiments are LEDs, advantaged for features such as low cost, long lifetimes, and compact packaging. However, other light sources, or some combination of LED and other types of light sources can alternately be used. White or polychromatic light can be provided by LEDs that have the same spectral output or can be provided by a combination of multiple LEDs or other light sources of different color.

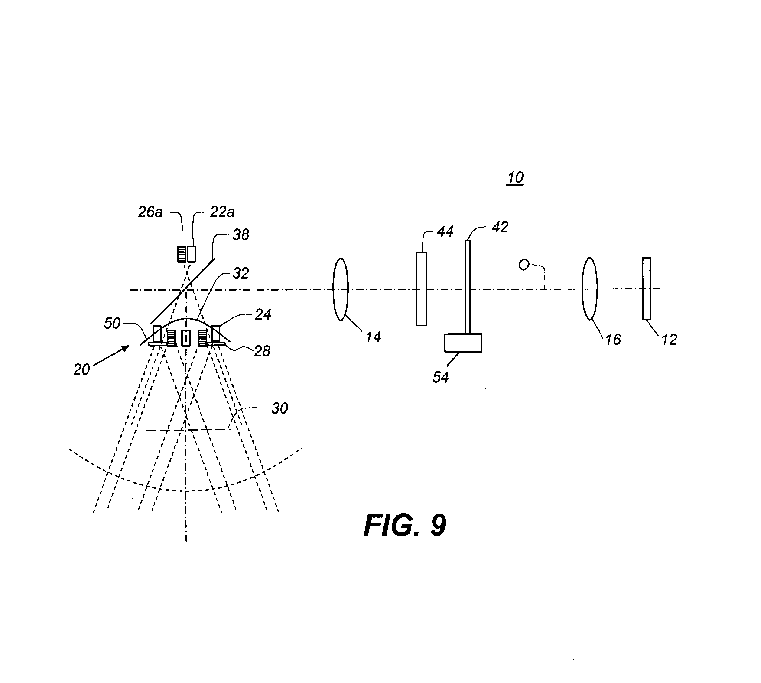

Because of the limited distance that is available for the illumination to propagate and spread, it can be difficult to provide suitable uniformity over the field that contains the tooth or other object to be imaged. With LEDs or other light sources positioned about the optical axis, the center of the field may be under-illuminated, particularly where the tooth or other object is relatively close to the light sources. The schematic diagram of Figure 9 shows an alternate embodiment that helps to correct this type of condition by adding illumination along the center of the field. This arrangement is particularly useful where polarized white light and UV light sources are used.

Referring to Figure 9, a polarization beam splitter 38 is used for folding the optical path. For diagnostic imaging, one or more additional white LEDs 22a direct light through polarization beam splitter 38 to supplement the illumination at the center of the field. For fluorescence imaging, one or more additional UV LEDs 26a are energized to direct light at the center. The transmission axis of polarization beam splitter 38 is in parallel to the transmission axis of polarizers 28. Light returning through aperture 32 goes to collection optics as with other solutions.

Control logic processor 40, shown in Figure 2 and used in embodiments of Figures 4 -6 and 9, can perform the needed image processing for observation and diagnostic imaging, including FIRE image processing. Optionally, the image acquisition and processing can be performed by an external computer or other logic processor, including a networked processor. Images obtained can be displayed immediately as well as uploaded and stored for subsequent processing and display.

By providing different types of illumination in a compact package, embodiments of the present invention simplify the workflow for obtaining a FIRE image by the technician. For example, to begin an imaging session, the technician enables the observational imaging mode, positioning intra-oral imaging apparatus 10 in the patient's mouth until it is near a tooth for which a FIRE image is needed. During this positioning phase, the technician observes display 48 (Figure 2). White LEDs 22 (Figure 4) are energized for each in a continuous series of images. Once the apparatus is positioned as desired, the technician enters the command to obtain a FIRE image, using a pushbutton control in one embodiment. In rapid succession, the UV LEDs 26 and polarized white LEDs 24 are energized (in either order) so that the FIRE image can be obtained.

The apparatus and methods of the present invention can be used to obtain a set of one or more images for informational and diagnostic use. The same image collection optics and image detector 12 are used for each image type, whether using unpolarized polychromatic light, polarized polychromatic light, or UV light.

The invention has been described in detail with particular reference to a presently preferred embodiment, but it will be understood that variations and modifications can be effected within the spirit and scope of the invention. For example, collection optics for imaging, schematically represented by two lenses 14 and 16 in embodiments of Figures 2, 4-6, and 9, can include any suitable number of light-directing components. Components such as long-pass filter 42 and analyzer 44 can alternately be switched into or out of the optical path as needed for the different imaging functions, using a galvonometric actuator 54 (Figure 9) or other actuator device. A number of possible mechanisms can be used for operator instruction entry and mode selection. Polarized light sources can be used in place of white light sources paired with polarizers 28. The presently disclosed embodiments are therefore considered in all respects to be illustrative and not restrictive. The scope of the invention is indicated by the appended claims, and all changes that come within the meaning and range of equivalents thereof are intended to be embraced therein.

### PARTS LIST

10. Intra-oral imaging apparatus
12. Detector
14, 16. Lens
20. Illumination system
22, 22a. White LED
24. White LED
26, 26a. UV LED
28. Polarizer
30, 34. Illumination field
32. Aperture
36. Fold mirror
38. Polarization beam splitter
40. Control logic processor
42. Long-pass filter
44. Analyzer
46. Mode switch
48. Display
50. Reflector
52. Switch
54. Actuator
α1, α2. Angle
D1, D2. Distance
O. Optical axis

## Claims

1. An intra-oral imaging apparatus comprising:
an illumination system disposed to direct light symmetrically about an optical axis and comprising:
a first array comprising a plurality of polychromatic light sources wherein the first array of polychromatic light sources are disposed about the optical axis to direct light over a first range of angles;
a second array comprising a plurality of polychromatic light sources wherein the second array of polychromatic light sources are disposed about the optical axis and wherein each light source of the second array is paired with a polarizer to direct light over a second range of angles that is narrower than the first range of angles; and
a third array comprising a plurality of ultraviolet light sources disposed about the optical axis to direct the light over the second range of angles; and
collection optics disposed along an optical path to direct reflected light to a detector that is disposed to form image data from the reflected light, the collection optics comprising:
an analyzer having its transmission axis oriented orthogonally to the polarizer to reduce specular reflection;
a long pass filter disposed to block reflected ultraviolet light from the third array light sources; and
an imaging lens system.

2. The intra-oral imaging apparatus of claim 1 further comprising an actuator that positions one or both the analyzer and the long pass filter in place for obtaining an image when the second array of polychromatic sources is energized.

3. The intra-oral imaging apparatus of claim 1 wherein each light source in the second array lies outside the first array of light sources with respect to the optical axis.

4. The intra-oral imaging apparatus of claim 1 wherein each light source in the third array lies outside the first array of light sources with respect to the optical axis.

5. The intra-oral imaging apparatus of claim 1 wherein at least one light source in at least one of the first, second, or third arrays of light sources is a light-emitting diode.

6. The intra-oral imaging apparatus of claim 1 wherein the first range of angles is within 80 degrees.

7. The intra-oral imaging apparatus of claim 1 further comprising a polarization beamsplitter disposed to redirect the optical axis.

8. An intra-oral imaging apparatus comprising:
an illumination system directing light symmetrically about an optical axis, the illumination system comprising:
a first array including a plurality of polychromatic light sources wherein the first array is disposed about the optical axis to direct light over a first range of angles;
a second array including a plurality of polychromatic light sources wherein the second array is disposed about the optical axis and wherein each of the polychromatic light sources is paired with a polarizer to direct light over a second range of angles that is narrower than the first range of angles;
a third array including a plurality of ultraviolet light sources disposed to direct the light over the second range of angles; and
a reflector disposed to redirect at least the light from the second and third arrays toward a predefined field;
a polarization beamsplitter disposed to fold the optical axis;
at least one additional light source disposed to direct light through the polarization beamsplitter and along the optical axis; and
collection optics disposed along the folded optical axis to direct reflected light to a detector that is disposed to form image data from the reflected light, the collection optics comprising:
an analyzer having its polarization transmission axis oriented orthogonally relative to the polarizer to reduce specular reflection;
an emission filter that is disposed to block reflected ultraviolet light from the third array light source; and
an imaging lens system.

9. The intra-oral imaging apparatus of claim 10 wherein the at least one additional light source is an ultraviolet light source.

10. The intra-oral imaging apparatus of claim 10 wherein the at least one additional light source is a polychromatic light source.

11. The intra-oral imaging apparatus of claim 10 wherein one or more of the light sources is a light-emitting diode.

12. An illumination system for an intra-oral imaging apparatus comprising:
a first array comprising a plurality of polychromatic light sources arranged radially about an optical axis;
a second array comprising a plurality of polychromatic light sources and polarizers arranged radially about the optical axis;
a third array comprising a plurality of ultraviolet light sources arranged radially about the optical axis; and
at least one additional polychromatic light source and at least one additional ultraviolet light source, both additional light sources directing light through a polarization beamsplitter and along the optical axis

13. The illumination system of claim 14 further comprising a reflector that is disposed between the first, second, and third arrays and the polarization beamsplitter.

14. A method for obtaining a set of intra-oral images, comprising:
energizing a first array of polychromatic light sources, obtaining one or more images, and displaying the one or more images during positioning of an intra-oral imaging apparatus in the mouth of a patient; and
energizing a second array of polychromatic light sources and a third array of ultraviolet light sources in response to an operator instruction, obtaining additional image data and storing the resulting image data in a computer-readable storage medium for processing.

15. The method of claim 14 further comprising combining the additional image data to form a diagnostic image and displaying the diagnostic image.
